# DEMANDE DE BREVET EUROPEEN

(11) **EP 2 338 463 A1**
(43) Date de publication de la demande: **29.06.2011**
(21) Numéro de dépôt: 10195262.0
(22) Date de dépôt: 15.12.2010
(51) Int. Cl.: A61K 8/06, A61K 8/31, A61K 8/34, A61Q 5/06, A61Q 5/10, A61K 8/22

(54) **Composition de coloration ou d'éclaircissement comprenant un corps gras et un polymere amphotère**

(30) Priorité: 22.12.2009 FR 0959391
(71) Demandeur: L'Oréal, 75008 Paris (FR)
(72) Inventeur: Ascione, Jean-Marc, 75003, Paris (FR); Cotteret, Jean, 78600, Maisons Laffite (FR)
(74) Mandataire: Fevrier, Murielle Françoise E.

(57) **Abrégé**

La présente invention a pour objet une composition de coloration ou d'éclaircissement des fibres kératiniques humaines, comprenant :
(a) au moins 25 % en poids d'un ou plusieurs corps gras ;
(b) un ou plusieurs polymères amphotères ;
(c) un ou plusieurs agents alcalinisants ; et
(d) du peroxyde d'hydrogène.

Elle concerne de plus un procédé de coloration ou d'éclaircissement la mettant en oeuvre.

Un autre objet de l'invention est constitué par des dispositifs à deux ou trois compartiments permettant d'obtenir, après mélange des compositions des compartiments, juste avant son application, une composition selon l'invention.

## Description

La présente invention a pour objet une émulsion de coloration ou d'éclaircissement des fibres kératiniques humaines sous forme d'émulsion inverse eau dans huile.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs colorants d'oxydation, habituellement une ou plusieurs bases d'oxydation éventuellement associées à un ou plusieurs coupleurs.

En général, les bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

On connaît également la coloration directe ou semi-permanente. Le procédé classiquement utilisé en coloration directe consiste à appliquer sur les fibres kératiniques des colorants directs qui sont des molécules colorées et colorantes, ayant une affinité pour les fibres, à laisser pauser, puis à les rincer.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques.

Ce type de procédé ne nécessite pas l'emploi d'un agent oxydant pour développer la coloration. Cependant, il n'est pas exclu d'en mettre en oeuvre afin d'obtenir avec la coloration, un effet d'éclaircissement. On parle alors de coloration directe ou semi-permanente en conditions éclaircissantes.

Les procédés de coloration permanente ou encore semi-permanente en conditions éclaircissantes, consistent donc à employer avec la composition tinctoriale, une composition aqueuse comprenant au moins un agent oxydant, en condition de pH alcalin dans la grande majorité des cas. Cet agent oxydant a entre autre pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

Il existe un besoin d'avoir une efficacité satisfaisante des produits d'éclaircissement et de coloration, notamment en terme de pouvoir éclaircissant ou d'intensité de coloration et/ou de sélectivité, tout en diminuant les effets néfastes liés à la présence simultanée d'agents alcalins et d'agents oxydants tels que le peroxyde d'hydrogène, notamment en diminuant la dégradation des fibres kératiniques et en réduisant les désagréments liés à l'odeur des agents alcalins mis en oeuvre comme l'ammoniaque et les amines.

On cherche donc à augmenter les effets des agents alcalins et/ou des agents oxydants tout en ayant une efficacité tinctoriale ou éclaircissante maximale sur les fibres kératiniques.

Ce but et d'autres sont atteints par la présente invention qui a donc pour objet une composition de coloration ou d'éclaircissement de fibres kératiniques humaines, comprenant :
(a) au moins 25 % en poids d'un ou plusieurs corps gras ne contenant pas de fonction acide carboxylique ;
(b) un ou plusieurs polymères amphotères ;
(c) un ou plusieurs agents alcalinisants ; et
(d) du peroxyde d'hydrogène.

Elle concerne également un procédé de traitement des fibres kératiniques humaines à partir de cette composition.

Lorsque cette composition est destinée à la coloration des fibres kératiniques, elle comprend en outre un ou plusieurs colorants d'oxydation et/ou un ou plusieurs colorants directs.

A l'inverse, lorsque la composition selon l'invention est destinée à la seule décoloration des fibres kératiniques, elle ne comprend pas de colorant direct ni de colorant d'oxydation (bases et coupleurs) ou bien, s'ils sont présents, leur teneur totale ne dépasse pas 0,005% en poids par rapport au poids de la composition. En effet, à une telle teneur, seule la composition serait teintée, c'est-à-dire qu'on n'observerait pas d'effet de coloration des fibres kératiniques.

L'invention a de même pour objet un dispositif à deux compartiments comprenant dans l'un, une première composition renfermant un ou plusieurs corps gras ne contenant pas de fonction acide carboxylique, un ou plusieurs agents alcalinisants, optionnellement un ou plusieurs colorants choisis parmi les colorants d'oxydation, les colorants directs ou leurs mélanges ; et dans l'autre, une deuxième composition renfermant le peroxyde d'hydrogène ; la première et/ou la seconde composition comprenant un ou plusieurs polymères amphotères..

L'invention concerne enfin un dispositif à trois compartiments comprenant dans l'un, une composition renfermant un ou plusieurs corps gras ne contenant pas de fonction acide carboxylique ; dans un autre, une composition renfermant un ou plusieurs agents alcalinisants et éventuellement un ou plusieurs colorants d'oxydation, un ou plusieurs colorants directs ou leurs mélanges ; et dans le dernier, une composition renfermant le peroxyde d'hydrogène ; au moins une de ces compositions comprenant un ou plusieurs polymères amphotères,.

Les compositions des différents dispositifs étant destinées à être mélangées pour donner la composition selon l'invention, juste avant l'application sur les fibres kératiniques humaines.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Comme indiqué auparavant, la composition selon l'invention comprend au moins 25 % en poids d'un ou plusieurs corps gras ne contenant pas de fonction carboxylique.

Par corps gras, on entend, un composé organique insoluble dans l'eau à température ordinaire (25°C) et à pression atmosphérique (760 mm de Hg ; soit 1,013.10⁵ Pa) (solubilité inférieure à 5% et de préférence à 1% encore plus préférentiellement à 0.1 %). Ils présentent dans leur structure au moins une chaine hydrocarbonée comportant au moins 6 atomes de carbone ou un enchaînement d'au moins deux groupements siloxane. En outre, les corps gras sont solubles dans des solvants organiques dans les mêmes conditions de température et de pression, comme par exemple le chloroforme, l'éthanol ou le benzène.

Dans le cadre de l'invention, par « corps gras ne contenant pas de fonction acide carboxylique », on désigne un corps gras ne contenant pas de groupement - COOH, ni de groupement -COO-.

Selon l'invention, les corps gras sont choisis parmi les composés liquides ou pâteux à température ambiante et à pression atmosphérique.

Plus particulièrement, les corps gras sont choisis parmi les alcanes inférieurs en C₆-C₁₆, les huiles non siliconées d'origine animale, végétale ou synthétique, les hydrocarbures d'origine minérale ou synthétique, les alcools gras, les esters d'acide gras et/ou d'alcool gras, les cires non siliconées, les silicones.

Il est rappelé qu'au sens de l'invention, les alcools et esters gras présentent plus particulièrement au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs, ces derniers comprennent de 6 à 16 atomes de carbone, sont linéaires ou ramifiés, éventuellement cycliques. A titre d'exemple, on peut citer l'hexane, l'undécane, le dodécane, le tridécane les isoparaffines comme l'isohexadécane et l'isodécane.

Comme huiles d'origine animale, végétale ou synthétique, utilisables dans la composition de l'invention, on peut citer par exemple :
- les huiles hydrocarbonées d'origine animale, telles que le perhydrosqualène.
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité.
- les hydrocarbures linéaires ou ramifiés, d'origine minérale ou synthétique de plus de 16 atomes de carbone, tels que les huiles de paraffine, volatiles ou non, et leurs dérivés, la vaseline, l'huile de vaseline, les polydécènes, le polyisobutène hydrogéné tel que Parléam^{®}.
- les huiles fluorées comme le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals; le perfluoro-1,2-diméthylcyclobutane; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.

Les alcools gras convenant à la mise en oeuvre de l'invention sont plus particulièrement choisis parmi les alcools saturés ou insaturés, linéaires ou ramifiés, comportant de 8 à 30 atomes de carbone. On peut citer par exemple l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

En ce qui concerne les esters d'acide gras et/ou d'alcools gras, avantageusement différents des triglycérides mentionnés ci-dessus ; on peut citer notamment les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

La cire ou les cires (non siliconées) sont choisies notamment parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les silicones utilisables dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE^{®} FZ 3109 commercialisée par la société UNION CARBIDE, de formule :

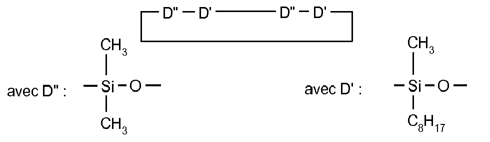

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2} R₃SiO_{1/2}, RSiO_{3/2} et SiO_{4/2}
   dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkylarylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

Plus particulièrement, les corps gras additionnels sont choisis parmi les composés liquides ou pâteux à température ambiante et à pression atmosphérique, de préférence liquide.

Les corps gras sont de préférence choisis parmi les alcanes inférieurs en C₆-C₁₆, les alcools gras, les esters d'acide gras, les esters d'alcool gras, les huiles en particulier les huiles non siliconées minérales, végétales ou synthétiques, les silicones.

De préférence, le corps gras est choisi parmi l'huile de vaseline, les polydécènes, les esters d'acides gras et/ou d'alcools gras liquides, les alcools gras liquides ou leurs mélanges.

La composition selon l'invention comprend au moins 25% de corps gras. Plus particulièrement, elle comprend une teneur en corps gras allant de 25 à 80% en poids, encore plus préférentiellement de 25 à 65% en poids, mieux de 30 à 55% en poids par rapport au poids de la composition.

La composition selon l'invention comprend un ou plusieurs polymères amphotères. Les polymères amphotères (ou zwitterioniques) utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire..Les polymères amphotères répondant à la définition donnée ci-dessus plus particulièrement préférés sont choisis parmi les polymères suivants :
1) les polymères comprenant à titre de monomères au moins un monomère dérivé d'un composé vinylique portant un groupement carboxylique tel que plus particulièrement l'acide acrylique, l'acide méthacrylique, l'acide maléïque, l'acide alpha-chloracrylique, et au moins un monomère basique dérivé d'un composé vinylique substitué contenant au moins un atome basique choisis notamment parmi
   a)les dialkyl aminoalkyl méthacrylates , les dialkyl aminoalkyl acrylates, les dialkyl aminoalkyl méthacrylamide et les dialkyl aminoalkyl acrylamide. De tels composés sont décrits dans le brevet américain n° 3 836 537.
   b)les sels de trialkyl aminoalkyl methacrylates et de trialkyl aminoalkyl acrylates, les sels de trialkylaminoalkylméthacrylamide et de trialkyl aminoalkyl acrylamide. On peut notamment citer le copolymère acide acrylique/chlorure d'acrylamidopropyl triméthyl ammonium proposé par la société Stockhausen sous la dénomination de Polymère W3794.. On peut aussi citer I les copolymères acide acrylique/chlorure d'acrylamidopropyl triméthyl ammonium/acrylamide proposés par la Société Nalco sous la dénomination Merquat 2001 et Merquat 2003.
(2) les polymères comportant des motifs dérivant :
   a) d'au moins un monomère choisi parmi les acrylamides ou les méthacrylamides substitués sur l'azote par un radical alkyle,
   b) d'au moins un co-monomère acide contenant un ou plusieurs groupements carboxyliques réactifs, et
   c) au moins comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.

Les co-monomères acides sont choisis plus particulièrement parmi les acides acrylique, méthacrylique, crotonique, itaconique, maléïque, fumarique ainsi que les monoesters d'alkyle ayant 1 à 4 atome de carbone des acides ou des anhydrides maléïque ou fumarique.

Les co-monomères basiques préférés sont des méthacrylates d'aminoéthyle, de butyl aminoéthyle, de N,N'-diméthylaminoéthyle, de N-tertio-butylaminoéthyle.
On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les copolymères comprenant à titre de monomères au moins un monomère dérivé d'un composé vinylique portant un groupement carboxylique tel que plus particulièrement l'acide acrylique, l'acide méthacrylique, l'acide maléïque, l'acide alpha-chloracrylique, et au moins un monomère de type sel de diallyl dialkyl ammonium , les groupes alkyle ayant de 1 à 6 atomes de carbone. De préférence le groupe alkyle est un groupe méthyle.

Parmi ces polymères, les copolymères comprenant à titre de monomères du chlorure de diméthyldiallylammonium et de l'acide acrylique éventuellement associés à de l'acrylamide sont paticulièrement préférés. On peut en particulier citer les composés proposés par la Société NALCO sous les dénominations Merquat 280, Merquat 295 , Merquat 3330, Merquat3331 et Merquat 3333.
(4) les polyamino amides réticulés et alcoylés partiellement ou totalement dérivant de poyaminoamides de formule générale : 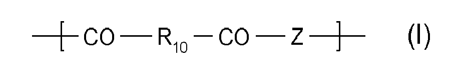 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 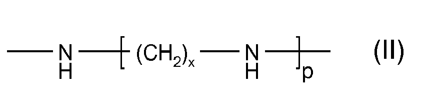 où x=2 et p=2 ou 3, ou bien x=3 et p=2 ce radical dérivant de la diéthylène triamine, de la triéthylène tétraamine ou de la dipropylène triamine;
   b) dans les proportions de 0 à 40 moles % le radical (II) ci-dessus, dans lequel x=2 et p=1 et qui dérive de l'éthylènediamine, ou le radical dérivant de la pipérazine : 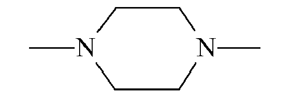
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium ou de potassium.
(5) les polymères comportant des motifs zwittérioniques de formule : 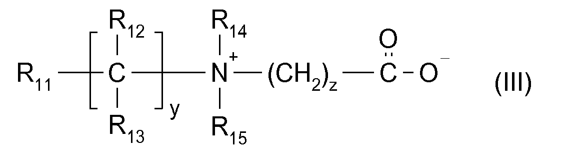 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.

A titre d'exemple, on peut citer le copolymère de méthacrylate de méthyle/diméthyl carboxyméthylammonio éthylméthacrylate de méthyle tel que le produit vendu sous la dénomination DIAFORMER Z301 par la société SANDOZ.
(6) les polymères dérivés du chitosane comportant des motifs monomères répondant aux formules suivantes : 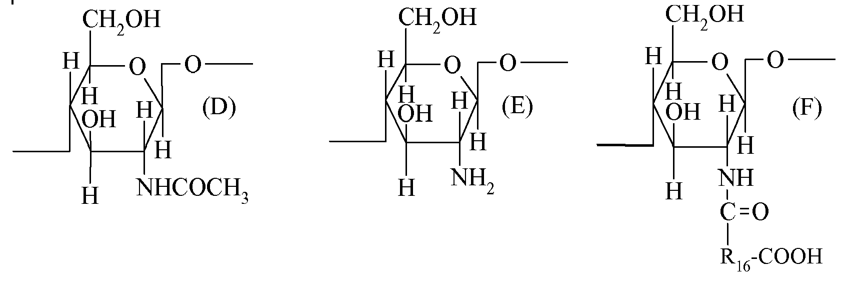 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 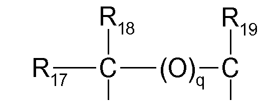 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, acoylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(7) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(8) Les polymères ayant des motifs répondant à la formule générale (IV) sont décrits dans le brevet français 1 400 366 : 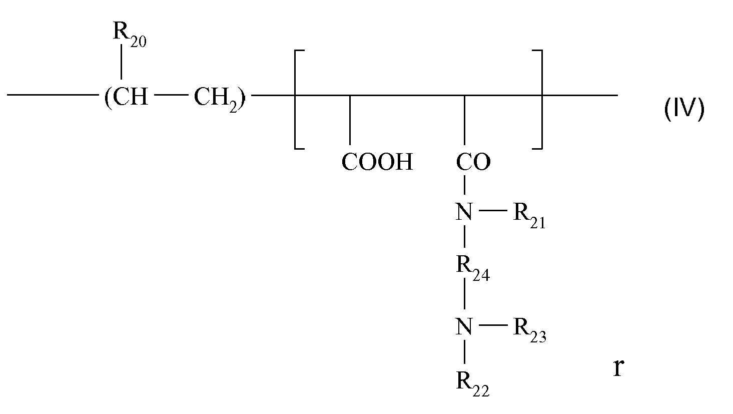 dans laquelle R₂₀ représente un atome d'hydrogène, un radical CH₃O CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂-, -CH₂-CH₂-CH₂- , - CH₂-CH(CH₃)- , R₂₂ ayant les significations mentionnées ci-dessus, ainsi que les homologues supérieurs de ces radicaux et contenant jusqu'à 6 atomes de carbone.
(9) Des polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (V)

      où D désigne un radical 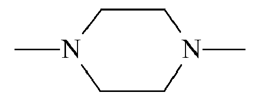 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI)

      où D désigne un radical 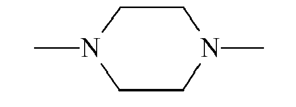 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonction carboxyle ou une ou plusieurs fonctions hydroxyle et bétaïnisées par réaction avec l'acide chloracétique ou du chloracétate de soude.
(10) les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux des familles (1) et (3) .

On préfèrera tout particulièrement les composés de la famille (1) et parmi ceux-ci le copolymère acide acrylique/chlorure d'acrylamidopropyl triméthyl ammonium.

Dans la composition de l'invention, le ou les polymères amphotères représente(nt) de préférence de 0,005 à 10% mieux de 0,05 à 5% et encore plus préférentiellement de 0,1 à 2% en poids, par rapport au poids total de la composition.

La composition comprend au moins un agent alcalinisant. Cet agent peut être choisi parmi les agents alcalins minéraux ou organiques ou hybrides ou leurs mélanges.

Le ou les agents alcalins minéraux sont de préférence choisis parmi l'ammoniaque, les carbonates ou bicarbonates alcalins comme les carbonates de sodium ou de potassium et les bicarbonates de sodium ou de potassium, les hydroxydes de sodium ou de potassium ou leurs mélanges.

Le ou les agents alcalins organiques sont de préférence choisis parmi les amines organiques dont le pKb à 25C est inférieur à 12, et de préférence inférieur à 10, encore plus avantageusement inférieur à 6. Il est à noter qu'il s'agit du pKb correspondant à la fonction de basicité la plus élevée.

A titre de composés hybrides on peut mentionner les sels des amines citées précédemment avec des acides comme l'acide carbonique, l'acide chlorhydrique.

Le ou les agents alcalins organiques sont par exemple choisis parmi les alcanolamines, les éthylènediamines oxyéthylénées et/ou oxypropylénées, les acides aminés et les composés de formule (VI) suivante : 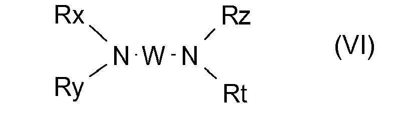 dans laquelle W est un reste alkylène en C₁-C₆ éventuellement substitué par un groupement hydroxyle ou un radical alkyle en C₁-C₆ Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles amines, le 1,3 diaminopropane, le 1,3 diamino 2 propanol, la spermine, la spermidine.

Par alcanolamine on entend une amine organique comprenant une fonction amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (VII) suivante : 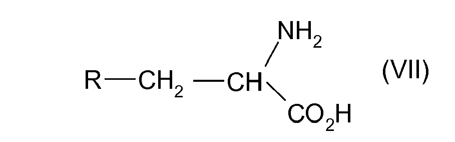 où R désigne un groupe choisi parmi :

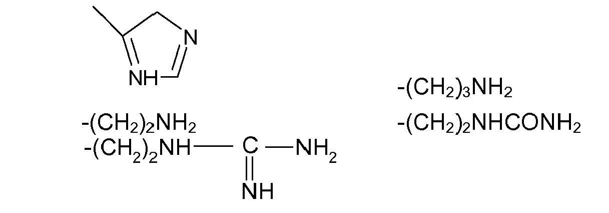

Les composés correspondants à la formule (VII) sont l'histidine, la lysine, l'arginine, l'ornithine, la citrulline.

L'amine organique peut être aussi choisie parmi les amines organiques de type hétérocycliques On peut en particulier citer, outre l'histidine déjà mentionnée dans les acides aminés, la pyridine, la pipéridine, l'imidazole, le triazole, le tétrazole, le benzimidazole.

L'amine organique peut être aussi choisie parmi les dipeptides d'acides aminés. A titre de dipeptides d'acides aminés utilisables dans la présente invention, on peut notamment citer la carnosine, l'anserine et la baleine

L'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino) methyl]amino)ethane-1-sulfonique.

A titre de composés hybrides on peut mentionner en particulier utiliser le carbonate de guanidine ou le chlorhydrate de monoéthanolamine.

La composition de l'invention contient de préférence une ou plusieurs alcanolamines, et/ou un ou plusieurs acides aminés basiques, plus avantageusement, une ou plusieurs alcanolamines. Encore plus préférentiellement l'amine organique est la monoéthanolamine.

De manière avantageuse, la composition selon l'invention présente une teneur en agent(s) alcalin(s), s'il(s) est(sont présent(s), allant de 0,01 à 30% en poids, de préférence de 0,1 à 20% en poids par rapport au poids de ladite composition.

La composition selon l'invention comprend également du peroxyde d'hydrogène.

La teneur en peroxyde d'hydrogène représente plus particulièrement de 0,1 à 20 % en poids, de préférence de 0,5 à 10% en poids, par rapport au poids de la composition.

Comme indiqué auparavant, la composition selon l'invention peut comprendre des colorants d'oxydation, des colorants directs ou leurs mélanges.

Les colorants d'oxydation sont en général choisis parmi les bases d'oxydation et les coupleurs

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-((β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-((β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE2359399 ; JP88-169571 ; JP05-63124 ; EP0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE3843892, DE4133957 et demandes de brevet WO94/08969, WO94/08970, FR-A-2733749 et DE19543988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préfére utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La composition selon l'invention peut éventuellement comprendre un ou plusieurs coupleurs conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

Les sels d'addition des bases d'oxydation et des coupleurs sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10% en poids par rapport au poids total de l'émulsion, et de préférence de 0,005 à 5% en poids par rapport au poids total de la composition.

La teneur en coupleur(s), s'il(s) est(sont) présent(s), représente chacun avantageusement de 0,0001 à 10% en poids par rapport au poids total de l'émulsion, et de préférence de 0,005 à 5% en poids par rapport au poids total de la composition.

Les colorants directs pouvant être utilisés sont par exemple les colorants synthétiques ou naturels, choisis parmi les espèces ioniques ou non ioniques, de préférence cationiques ou non ioniques.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges. En particulier, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Parmi les colorants directs benzéniques utilisables selon l'invention, on peut citer de manière non limitative les composés suivants :
- 1,4-diamino-2-nitrobenzène,
- 1-amino-2 nitro-4-ß- hydroxyéthylaminobenzène
- 1-amino-2 nitro-4-bis(ß-hydroxyéthyl)-aminobenzène
- 1,4-bis(ß -hydroxyéthylamino)-2-nitrobenzène
- 1-ß-hydroxyéthylamino-2-nitro-4-bis-((3-hydroxyéthylamino)-benzène
- 1-ß-hydroxyéthylamino-2-nitro-4-aminobenzène
- 1-ß-hydroxyéthylamino-2-nitro-4-(éthyl)(ß-hydroxyéthyl)-aminobenzène
- 1-amino-3-méthyl-4-ß-hydroxyéthylamino-6-nitrobenzène
- 1-amino-2-nitro-4-ß-hydroxyéthylamino-5-chlorobenzène
- 1,2-diamino-4-nitrobenzène
- 1-amino-2-p-hydroxyéthylamino-5-nitrobenzène
- 1,2-bis-(ß-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-b!s-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants suivants de formules (I) à (IV), et de préférence les composés de formules (II) , (III), (IV) et (IV') : 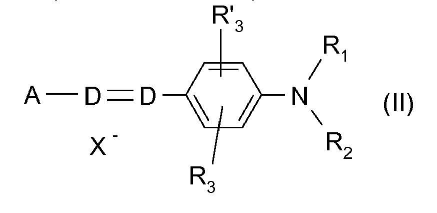 dans laquelle :
D représente un atome d'azote ou le groupement -CH, de préférence un atome d'azote,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄ , alcoxy en C₁-C₄ ou acétyloxy,
X - représente un anion de préférence choisi parmi le chlorure, le méthyl sulfate et l'acétate,
A représente un groupement choisi par les structures A₁ à A₁₈, et de préférence A1, A₄, A₇, A₁₃ et A₁₈, suivantes : 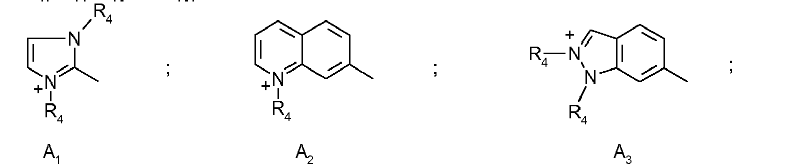 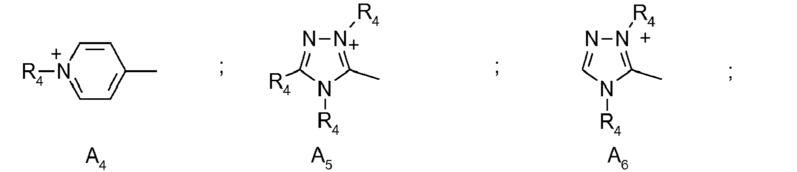 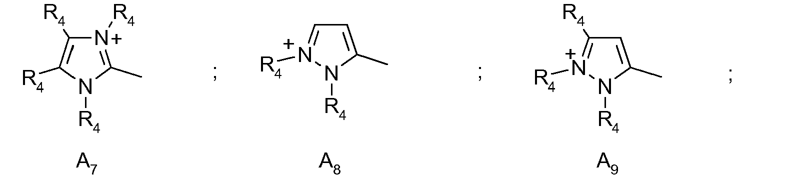 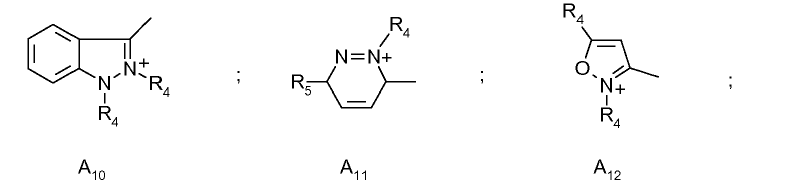 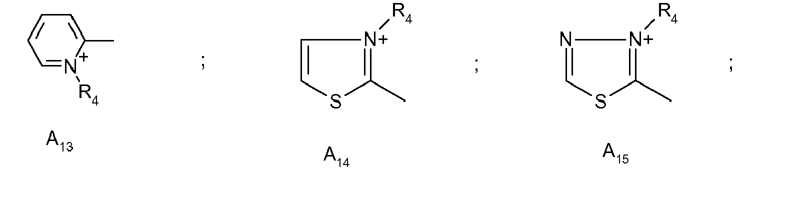 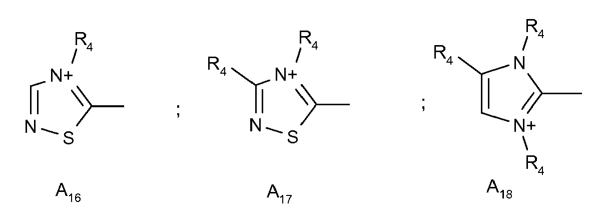 dans lesquelles R₄ représente un radical alkyle en C₁-C₄ pouvant être substitué par un radical hydroxyle et R₅ représente un radical alcoxy en C₁-C₄ ; 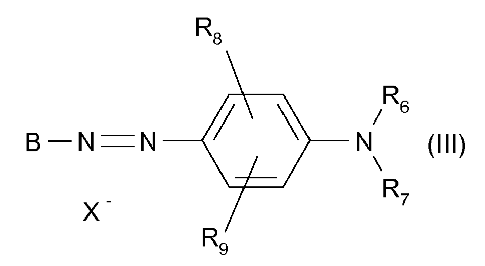 dans laquelle :
   R₆ représente un atome d'hydrogène ou un radical alkyle en C₁-C₄,
   R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
   R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
   X - représente un anion de préférence choisi parmi le chlorure, le méthyl sulfate et l'acétate,
   B représente un groupement choisi par les structures B1 à B6 suivantes : 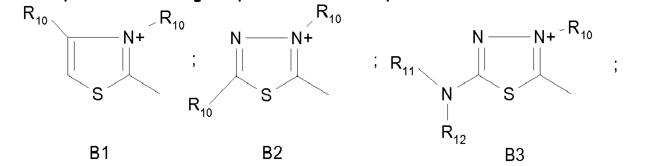 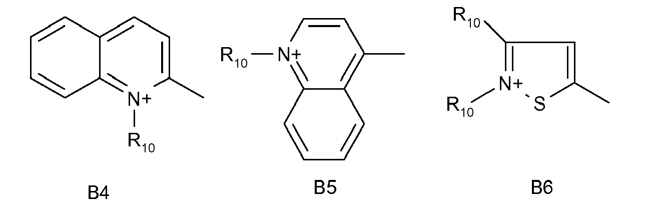 dans lesquelles R₁₀ représente un radical alkyle en C₁-C₄, R₁₁ et R₁₂, identiques ou différents, représentent un atome d'hydrogène ou un radical alkyle en C₁-C₄ ; 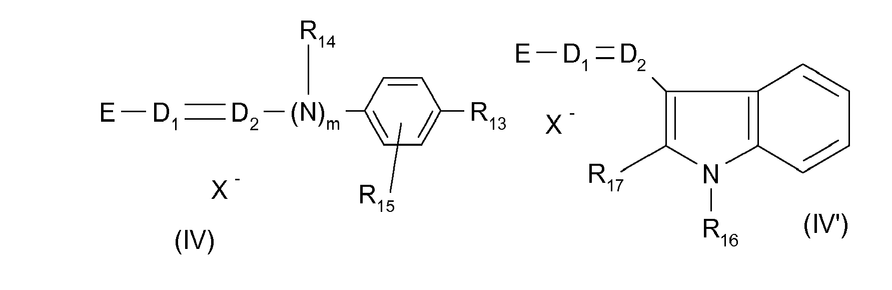 dans lesquelles :
      R₁₃ représente un atome d'hydrogène, un radical alcoxy en C₁-C₄, un atome d'halogène tel que le brome, le chlore, l'iode ou le fluor,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      R₁₆ et R₁₇, identiques ou différents, représentent un atome d'hydrogène ou un radical alkyle en C₁-C₄,
      D₁ et D₂, identiques ou différents, représentent un atome d'azote ou le groupement -CH,
      m = 0 ou 1,
      étant entendu que lorsque R₁₃ représente un groupement amino non substitué, alors D₁ et D₂ représentent simultanément un groupement -CH et m = 0,
      X - représente un anion de préférence choisi parmi le chlorure, le méthyl sulfate et l'acétate,
      E représente un groupement choisi par les structures E₁ à E₈, et de préférence E₁, E₂ et E₇, suivantes : 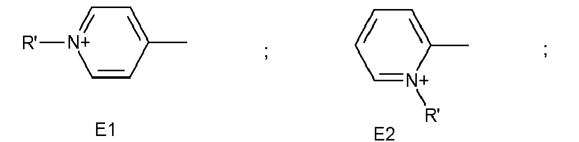 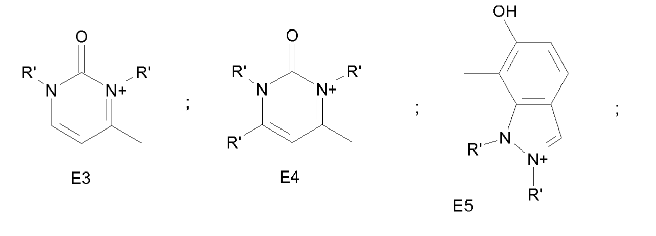 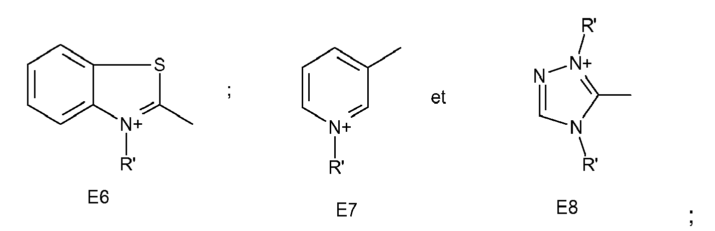 dans lesquelles R' représente un radical alkyle en C₁-C₄ ; lorsque m = 0 et que D₁ représente un atome d'azote, alors E peut également désigner un groupement de structure E9 suivante : 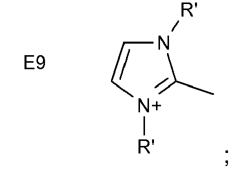 dans laquelle R' représente un radical alkyle en C₁-C₄.

         G-N=N-J (V)

         dans laquelle :
         le symbole G représente un groupement choisi parmi les structures G₁ à G₃ suivantes : 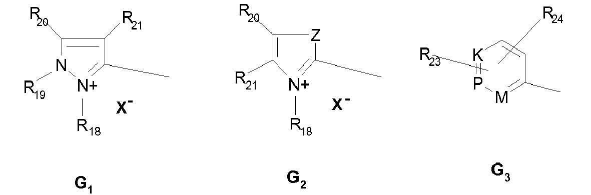 structures G₁ à G₃ dans lesquelles,
            R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
            R₁₉ désigne un radical alkyle en C₁-C₄ ou un radical phényle;
            R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄, alcoxy en C₁-C₄, ou NO₂;
            R₂₀ peut désigner en outre un atome d'hydrogène;
            Z désigne un atome d'oxygène, de soufre ou un groupement -NR₁₉;
            M représente un groupement -CH, -CR (R désignant alkyle en C₁-C₄),
            ou -NR₂₂(X⁻)ᵣ;
            K représente un groupement -CH, -CR (R désignant alkyle en C₁-C₄),
            ou -NR₂₂₍X⁻)ᵣ;
            P représente un groupement -CH, -CR (R désignant alkyle en C₁-C₄),
            ou -NR₂₂(X⁻)ᵣ; r désigne zéro ou 1;
            R₂₂ représente un atome O⁻, un radical alcoxy en C₁-C₄, ou un radical alkyle en C₁-C₄;
            R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
            X⁻ représente un anion de préférence choisi parmi le chlorure, l'iodure, le méthyl sulfate, l'éthyl sulfate, l'acétate et le perchlorate;
            sous réserve que,
            si R₂₂ désigne O⁻, alors r désigne zéro;
            si K ou P ou M désignent -N-alkyle C₁-C₄ X⁻ , alors R₂₃ ou R₂₄ est ou non différent d'un atome d'hydrogène;
            si K désigne -NR₂₂(X⁻)ᵣ , alors M= P= -CH, -CR;
            si M désigne -NR₂₂(X⁻)ᵣ , alors K= P= -CH, -CR;
            si P désigne -NR₂₂(X⁻)ᵣ , alors K= M et désignent -CH ou -CR;
            si Z désigne un atome de soufre avec R₂₁ désignant alkyle en C₁-C₄ , alors R₂₀ est différent d'un atome d'hydrogène;
            si Z désigne -NR₂₂ avec R₁₉ désignant alkyle en C₁-C₄ , alors au moins l'un des radicaux R₁₈, R₂₀ ou R₂₁ du groupement de structure G₂ est différent d'un radical alkyle en C₁-C₄;
            le symbole J représente :
            - (a) un groupement de structure J₁ suivante : 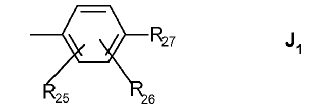 structure J₁ dans laquelle,
               R₂₅ représente un atome d'hydrogène, un atome d'halogène choisi parmi le chlore, le brome, l'iode et le fluor, un radical alkyle en C₁-C₄, alcoxy en C₁-C₄, un radical -OH, - NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
               R₂₆ représente un atome d'hydrogène, un atome d'halogène choisi parmi le chlore, le brome, l'iode et le fluor, un radical alkyle en C₁-C₄, alcoxy en C₁-C₄,
               ou forme avec R₂₇ ou R₂₈ un cycle à 5 ou 6 chaînons contenant ou non un ou plusieurs hétéroatomes choisis parmi l'azote, l'oxygène ou le soufre;
               R₂₇ représente un atome d'hydrogène, un radical -OH, un radical -NHR₂₈, un radical - NR₂₉R₃₀;
               R₂₈ représente un atome d'hydrogène, un radical alkyle en C₁-C₄, un radical monohydroxyalkyle en C₁-C₄, polyhydroxyalkyle en C₂-C₄, un radical phényle;
               R₂₉ et R₃₀, identiques ou différents, représentent un radical alkyle en C₁-C₄ , un radical monohydroxyalkyle en C₁-C₄, polyhydroxyalkyle en C₂-C₄;
            - (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
               et notamment un groupement de structure J₂ suivante : 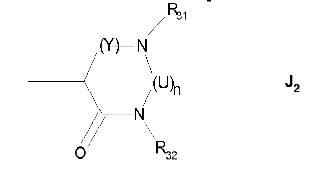 structure J₂ dans laquelle,
               R₃₁ et R₃₂, identiques ou différents, représentent un atome d'hydrogène, un radical alkyle en C₁-C₄, un radical phényle;
               Y désigne le radical -CO- ou le radical 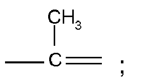 n = 0 ou 1, avec, lorsque n désigne 1, U désigne le radical -CO- .

Dans les structures (II) à (V) définies ci-dessus le groupement alkyle ou alcoxy en C₁-C₄ désigne de préférence méthyle, éthyle, butyle, méthoxy, éthoxy.

Parmi les composés de formules (II) et (IV), on préfère les composés suivants :

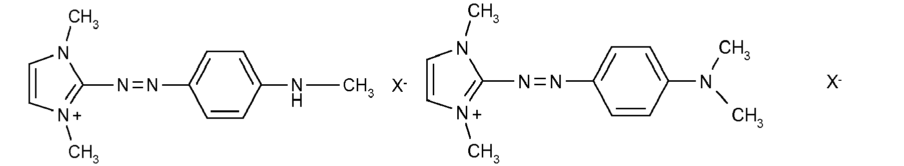

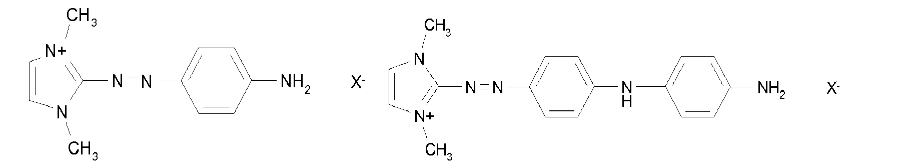

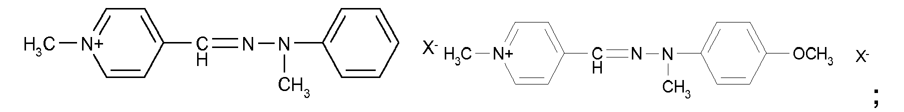

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99
   ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous :

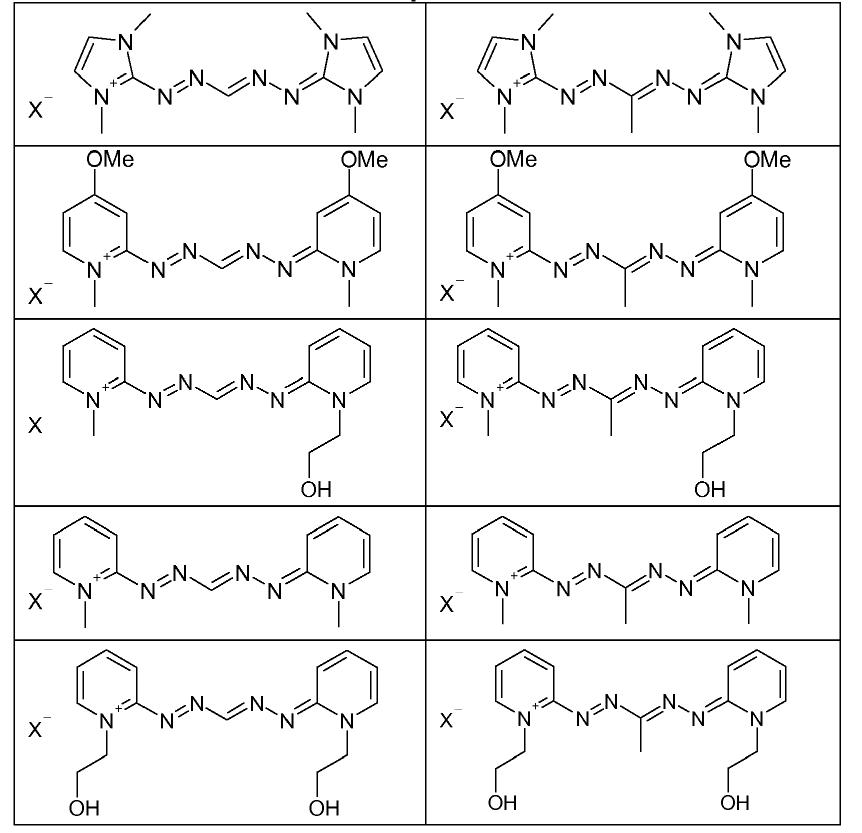

X⁻ représente un anion de préférence choisi parmi le chlorure, l'iodure, le méthyl sulfate, l'éthyl sulfate, l'acétate et le perchlorate.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, l'hématoxyline, l'hémateine, la brasiline, la brasiléine, les orcéines. On peut également utiliser les extraits ou décoctions contenant ces colorants naturels et notamment les cataplasmes ou extraits à base de henné.

Les colorants directs peuvent être des colorants monochromophoriques (c'est-à-dire ne comprenant qu'un seul colorant) ou polychromophoriques, de préférence di- ou tri- chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement interrompue par au moins un groupement ammonium quaternaire substitué par deux groupements alkyle en C₁-C₁₅ éventuellement substitués ; le bras de liaison ne comprenant pas de groupement nitro, nitroso ou peroxo.

Si les hétérocycles ou noyaux aromatiques (phényle ou naphtyle) sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en C₁-C₂ ; un radical hydroxyalcoxy en C₂-C₄ ; un radical amino ; un radical amino substitué par un ou deux radicaux alkyle, identiques ou différents, en C₁-C₄ éventuellement porteurs d'au moins un groupement hydroxyle.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en C₁-C₆, un noyau phényle éventuellement substitué, ou d'au moins un groupement N(R')₂ avec R' identiques ou non, représentant un atome d'hydrogène, un radical alkyle éventuellement substitué en C₁-C₆, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

La liaison entre le bras de liaison, tel que défini précédemment, et chaque chromophore se fait en général au moyen d'un hétéroatome substituant le noyau phényle ou napthyle ou au moyen de l'atome d'azote quaternisé de l'hétérocycle cationique.

Le colorant peut comprendre des chromophores identiques ou non.

A titre d'exemples de tels colorants, on pourra notamment se reporter aux demandes de brevets EP1637566, EP1619221, EP1634926, EP1619220, EP1672033, EP1671954, EP1671955, EP1679312, EP1671951, EP167952, EP167971, WO 06/063866, WO06/063867, WO06/063868, WO06/063869, EP1408919, EP1377264, EP1377262, EP1377261, EP1377263, EP1399425, EP1399117, EP1416909, EP 1399116, EP1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Lorsqu'ils sont présents, le ou les colorants directs représentent plus particulièrement de 0,0001 à 10% en poids du poids total de la composition, et de préférence de 0,005 à 5% en poids.

La composition selon l'invention peut également comprendre un ou plusieurs tensioactifs.

De préférence, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylarylpolyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamidesulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène et/ou de propylène compris entre 1 et 100, de préférence entre 2 et 50 de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés comprenant de 1 à 100 moles d'oxyde d'éthylène ; les esters d'acides en C₈-C₃₀, saturés ou non, linéaires ou ramifiés, et de sorbitol polyoxyéthylénés comprenant de 1 à 100 moles d'oxyde d'éthylène.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif éventuellement présent dans la composition est un tensioactif non ionique.

La teneur en tensioactifs dans la composition représente plus particulièrement de 0,1 à 50% en poids, de préférence de 0,5 à 30 % en poids par rapport au poids de la composition.

La composition peut également renfermer divers adjuvants utilisés classiquement dans les compositions pour la coloration ou l'éclaircissement des cheveux, tels que des polymères anioniques, cationiques, non ioniques, ou leurs mélanges ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20 % en poids par rapport au poids de la composition.

La composition peut comprendre une ou plusieurs silices pyrogénées.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

Lorsqu'elle est présente, la silice pyrogénée représente de 1 à 30 % en poids par rapport au poids de la composition.

La composition peut également comprendre un ou plusieurs agents épaississants organiques.

Ces agents épaississants peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères comprenant des zones hydrophiles, et des zones hydrophobes à chaîne grasse (alkyle, alcényle comprenant au moins 10 atomes de carbone) capables, dans un milieu aqueux, de s'associer réversiblement entre eux ou avec d'autres molécules.).

Selon un mode de réalisation particulier, l'épaississant organique est choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique, et de préférence parmi les épaississants cellulosiques avec en particulier l'hydroxyéthycellulose.

La teneur en agent(s) épaississant(s) organique(s), s'ils sont présents, varie habituellement de 0,01 % à 20 % en poids, par rapport au poids de la composition, de préférence de 0,1 à 5 % en poids.

La composition selon l'invention peut comprendre de l'eau et /ou un ou plusieurs solvants organiques.

A titre de solvant organique, on peut par exemple citer les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol le butylèneglycol, le dipropylèneglycol et le propylèneglycol; les alcools aromatiques tels que l'alcool benzylique, l'alcool phényléthylique ; ; les polyols à plus de deux fonctions hydroxyles tels que le glycérol ; les éthers de polyol tels que, par exemple, les éthers monométhylique, monoéthylique et monobutylique d'éthylèneglycol, le propylèneglycol ou ses éthers tels que, par exemple, le monométhyléther de propylèneglycol,; ainsi que les alkyléthers de diéthylèneglycol, notamment en C₁-C₄, comme par exemple, le monoéthyléther ou le monobutyléther du diéthylèneglycol, seuls ou en mélange.

Les solvants organiques, quand ils sont présents, représentent généralement entre 1 et 40% en poids par rapport au poids total de la composition tinctoriale, et de préférence entre 5 et 30% en poids par rapport au poids total de la composition tinctoriale.

De préférence la composition de l'invention contient de l'eau, par exemple entre 10 à 70%, mieux de 20 à 55% du poids total de la composition.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Avantageusement, la composition selon l'invention se présente sous la forme d'un gel ou d'une crème.

Le pH de la composition selon l'invention est généralement compris entre 3 et 12, de préférence entre 5 et 11. Préférentiellement entre 7 et 11 bornes comprises.

Il peut être ajusté à la valeur désirée au moyen d'agents acidifiants ou alcalinisants habituellement utilisés en teinture des fibres kératiniques

Les agents alcalinisants sont par exemple ceux décrits précédemment.

Parmi les agents acidifiants, on peut citer, à titre d'exemple, des acides minéraux ou organiques comme l'acide chlorhydrique, l'acide ortho-phosphorique, des acides carboxyliques comme l'acide tartrique, l'acide citrique, l'acide lactique, ou des acides sulfoniques.

La composition de l'invention peut être obtenue par mélange d'au moins deux compositions différentes, voire trois ou éventuellement plus de trois compositions différentes. Une ou plusieurs des compositions conduisant par mélange à la composition de l'invention peuvent être anhydre. A noter que la composition selon l'invention est préparée juste avant son application sur les fibres kératiniques humaines.

Selon une première variante, la composition selon l'invention est obtenue en mélangeant une première composition comprenant un ou plusieurs corps gras, un ou plusieurs agents alcalinisants, et éventuellement un ou plusieurs colorants d'oxydation, colorants directs ou leurs mélange avec une deuxième composition comprenant le peroxyde d'hydrogène ; la première et/ou la seconde composition comprenant un ou plusieurs polymères amphotères.

Selon une deuxième variante de l'invention, la composition selon l'invention est obtenue en mélangeant une première composition comprenant un ou plusieurs corps gras, une deuxième composition comprenant un ou plusieurs agents alcalinisants et éventuellement un ou plusieurs colorants d'oxydation, colorants directs ou leurs mélanges et une troisième composition comprenant le peroxyde d'hydrogène ; la première et/ou la seconde et/ou la troisième composition comprenant un ou plusieurs polymères amphotères.

Les ingrédients des compositions précitées et leurs teneurs sont déterminés en fonction des caractéristiques détaillées auparavant pour la composition finale selon l'invention.

Dans chacune des variantes précitées, la composition comprenant le peroxyde d'hydrogène est de préférence une composition aqueuse qui comprend plus de 5 % en poids d'eau, de préférence plus de 10% en poids d'eau, et de manière encore plus avantageuse plus de 20% en poids d'eau.

Elle peut également comprendre un ou plusieurs solvants organiques choisis parmi ceux listés auparavant ; ces derniers représentant plus particulièrement, lorsqu'ils sont présents, de 1 à 40% en poids par rapport au poids de la composition oxydante, et de préférence de 5 à 30% en poids.

La composition oxydante comprend également de manière préférée, un ou plusieurs agents acidifiants. Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition oxydante, lorsqu'elle est aqueuse, est inférieur à 7.

De préférence, la concentration en peroxyde d'hydrogène varie, plus particulièrement, de 0,1 à 50%, plus particulièrement entre 0,5 et 20%, et encore plus préférentiellement entre 1 et 15% en poids par rapport au poids de la composition oxydante.

Le procédé de coloration selon l'invention consiste donc à appliquer la composition selon l'invention, sur les fibres kératiniques humaines, sèches ou humides. La température durant le procédé est classiquement comprise entre la température ambiante (entre 15 à 25°C) et 80°C, de préférence entre la température ambiante et 60°C.

Après un temps de pause d'une minute à une heure, de préférence de 5 minutes à 30 minutes, les fibres kératiniques sont éventuellement rincées à l'eau, subissent éventuellement un lavage avec un shampooing suivi d'un rinçage à l'eau, avant d'être séchées ou laissées sécher.

L'invention concerne aussi un dispositif à deux compartiments renfermant dans l'un, une première composition comprenant un ou plusieurs corps gras, un ou plusieurs agents alcalinisants et éventuellement un ou plusieurs colorants d'oxydation et/ou colorants directs ; dans l'autre, une deuxième composition comprenant du peroxyde d'hydrogène; la première et/ou la seconde composition comprenant un ou plusieurs polymères amphotères, les compositions des compartiments étant destinées à être mélangées pour donner la composition selon l'invention, juste avant l'application sur les fibres kératiniques humaines.

L'invention concerne enfin un dispositif à trois compartiments renfermant dans l'un, une première composition comprenant un ou plusieurs corps gras ; dans un autre, une deuxième composition comprenant un ou plusieurs agents alcalinisants et éventuellement un ou plusieurs colorants d'oxydation et/ou colorants directs ; et dans le dernier, une troisième composition comprenant du peroxyde d'hydrogène ; la première et/ou la seconde et/ou la troisième composition comprenant un ou plusieurs polymères amphotères ; les compositions des trois compartiments étant destinées à être mélangées pour donner la composition selon l'invention, juste avant l'application sur les fibres kératiniques humaines.

Les exemples suivants servent à illustrer l'invention sans toutefois présenter un caractère limitatif.

### EXEMPLES

On prépare les compositions suivantes (les quantités sont exprimées en g% de matières actives)

### Composition 1

| | |
|---|---|
| Disteardimonium hectorite (Bentone 38 VCG) | 3 |
| Octyldodécanol | 11,5 |
| Distéarate de glycol | 8 |
| Huile de vaseline | 64,5 |
| Carbonate de propylène | 1 |
| Laureth-2 | 1 |
| Polysorbate 21 | 11 |

### Composition 2

| | |
|---|---|
| Pentasodium pentetate | 1 |
| Métabisulfite de sodium | 0,7 |
| Monoéthanolamine | 14,5 |
| Toluène-2,5-diamine | 2,25 |
| Chlorhydrate de 2,4-diaminophénoxyéthanol | 0,05 |
| Résorcinol | 2 |
| m-aminophénol | 0,36 |
| Hydroxyéthylcellulose (Natrosol 250 HHR , Aqualon) | 1,5 |
| Hexylène glycol | 3 |
| Dipropylène glycol | 3 |
| Ethanol | 8,25 |
| Propylèneglycol | 6,2 |
| Acide ascorbique | 0,25 |
| eau | Qsp 100 |

### Compositions 3 et 3'

| | Comp 3 | Comp 3' |
|---|---|---|
| Pentasodium pentetate | 0,15 | 0,15 |
| Peroxyde d'hydrogène (solution aqueuse à 50 %) | 12 | 12 |
| Stannate de sodium | 0,04 | 0,04 |
| Acide phosphorique | Qs pH 2.2 | Qs pH 2.2 |
| Pyrophosphate tétrasodique | 0,03 | 0,03 |
| Huile de vaseline | 20 | 20 |
| Copolymère acide acrylique/chlorure d'acrylamidopropyl triméthyl ammonium proposé par la société Stockhausen sous la dénomination de Polymère W3794 | 0,3 | |
| Copolymère chlorure de diméthyldiallylammonium et de l'acide acrylique proposé par la Société NALCO sous la dénomination Merquat 280 | | 0,3 |
| Glycérine | 0,5 | 0,5 |
| Alcool cétylstéarylique (C₁₆/C₁₈ 30/70 - NAFOL 1618F) | 8 | 8 |
| Alcool cétylstéarylique oxyéthyléné (33 OE) | 3 | 3 |
| Amide d'acides de colza oxyethylene (4 OE) | 1,2 | 1,2 |
| Vitamine E : DL-α-tocophérol | 0,1 | 0,1 |
| eau | Qsp 100 | Qsp 100 |

### Mode d'application

Les compositions détaillées ci-dessus sont mélangées au moment de l'emploi dans les proportions suivantes :
* 10 g de la composition 1
* 4 g de la composition 2
* 16 g de la composition 3 ou 3'.

Le mélange résultant est ensuite appliqué sur des mèches de cheveux naturels à 90% de blancs, à raison de 10 g de mélange pour 1 g de cheveux.

Le mélange est laissé à température ambiante pendant 30 minutes.

Les cheveux sont ensuite rincés, lavés avec un shampooing standard et séchés.

On obtient des mèches châtain clair (évaluation visuelle).Les cheveux présentent un excellent toucher.

## Revendications

1. Composition de traitement des fibres kératiniques humaines, **caractérisée en ce qu'**elle comprend:
(a) au moins 25 % en poids d'un ou plusieurs corps gras ne contenant pas de fonction carboxylique ;
(b) un ou plusieurs polymères amphotères;
(c) un ou plusieurs agents alcalinisants ; et
(d) du peroxyde d'hydrogène.

2. Composition selon la revendication précédente, **caractérisée en ce que** le ou les corps gras sont choisis parmi les alcanes inférieurs en C₆-C₁₆, les huiles non siliconées d'origine animale, végétale ou synthétique, les hydrocarbures d'origine minérale ou synthétique, les alcools gras, les esters d'acide gras et/ou d'alcool gras, les cires non siliconées, les silicones.

3. Composition selon l'une quelconque des revendications 1 ou 2, **caractérisée en ce que** le ou les corps gras sont choisis parmi les composés liquides ou pâteux, et de préférence liquides à température ambiante et à pression atmosphérique.

4. Composition selon l'une quelconque des revendications précédentes dans laquelle le corps gras est choisi parmi les alcanes inférieurs en C₆-C₁₆, les huiles non siliconées d'origine végétale ou synthétique, les hydrocarbures d'origine minérale ou synthétique, les alcools gras, les esters d'acide gras et/ou d'alcool gras.

5. Composition selon l'une quelconque des revendications précédentes dans laquelle le corps gras est choisi parmi l'huile de vaseline, les polydécènes, les esters d'acides gras et/ou d'alcools gras liquides, les alcools gras liquides ou leurs mélanges.

6. Composition selon l'une quelconque des revendications précédentes, **caractérisée en ce que** la teneur en corps gras varie de 25 à 80 % en poids, de préférence de 25 et 65% en poids, mieux de 30 à 55% en poids par rapport au poids de la composition.

7. Composition selon l'une quelconque des revendications précédentes, **caractérisée en ce que** le ou les polymères sont choisis parmi les composés suivants :
1) les polymères comprenant à titre de monomères au moins un monomère dérivé d'un composé vinylique portant un groupement carboxylique de préférence l'acide acrylique, l'acide méthacrylique, l'acide maléïque, l'acide alpha-chloracrylique, et au moins un monomère basique dérivé d'un composé vinylique substitué contenant au moins un atome basique choisis parmi
a)les dialkyl aminoalkyl méthacrylates , les dialkyl aminoalkyl acrylates, les dialkyl aminoalkyl méthacrylamide et les dialkyl aminoalkyl acrylamide.
b)les sels de trialkyl aminoalkyl methacrylates et de trialkyl aminoalkyl acrylates, les sels de trialkylaminoalkylméthacrylamide et de trialkyl aminoalkyl acrylamide.
(2) les polymères comportant des motifs dérivant :
a) d'au moins un monomère choisi parmi les acrylamides ou les méthacrylamides substitués sur l'azote par un radical alkyle contenant de préférence de 2 à 12 atomes de carbone,
b) d'au moins un comonomère acide contenant un ou plusieurs groupements carboxyliques réactifs de préférence choisis parmi les acides acrylique, méthacrylique, crotonique, itaconique, maléïque, fumariqu ainsi que parmi les monoesters d'alkyle ayant 1 à 4 atome de carbone des acides ou des anhydrides maléïque ou fumarique.
c) au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
(3) les copolymères comprenant à titre de monomères au moins un monomère dérivé d'un composé vinylique portant un groupement carboxylique tel que plus particulièrement l'acide acrylique, l'acide méthacrylique, l'acide maléïque, l'acide alpha-chloracrylique, et au moins un monomère de type sel de diallyl dialkyl ammonium , les groupes alkyle ayant de 1 à 6 atomes de carbone.
(4) les polyamino amides réticulés et alcoylés partiellement ou totalement dérivant de poyaminoamides de formule générale : 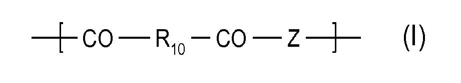 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
a) dans les proportions de 60 à 100 moles %, le radical 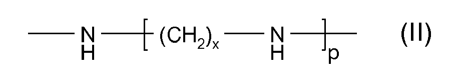 où x=2 et p=2 ou 3, ou bien x=3 et p=2 ce radical dérivant de la diéthylène triamine, de la triéthylène tétraamine ou de la dipropylène triamine;
b) dans les proportions de 0 à 40 moles % le radical (II) ci-dessus, dans lequel x=2 et p=1 et qui dérive de l'éthylènediamine, ou le radical dérivant de la pipérazine : 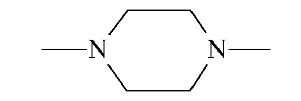
c) dans les proportions de 0 à 20 moles % le radical -NH-(CH2)6-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
(5) les polymères comportant des motifs zwittérioniques de formule : 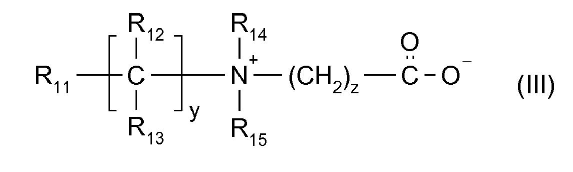 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
(6) les polymères dérivés du chitosane comportant des motifs monomères répondant aux formules suivantes : 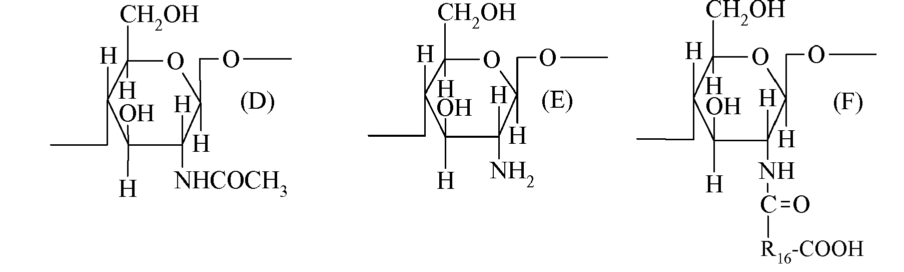 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 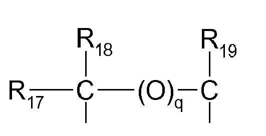 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, acoylthio, sulfonique, un reste alcoylthio dont le groupe alcoyle porte un reste amino, l'un au moins des radicaux R₁₇, R₁₈ et R₁₉ étant dans ce cas un atome d'hydrogène ;
ou si q=1, R₁₇, R₁₈ et R₁₉ représentent chacun un atome d'hydrogène, ainsi que les sels formés par ces composés avec des bases ou des acides.
(7) Les polymères dérivés de la N-carboxyalkylation du chitosane
(8) Les polymères ayant des motifs répondant à la formule générale (IV): 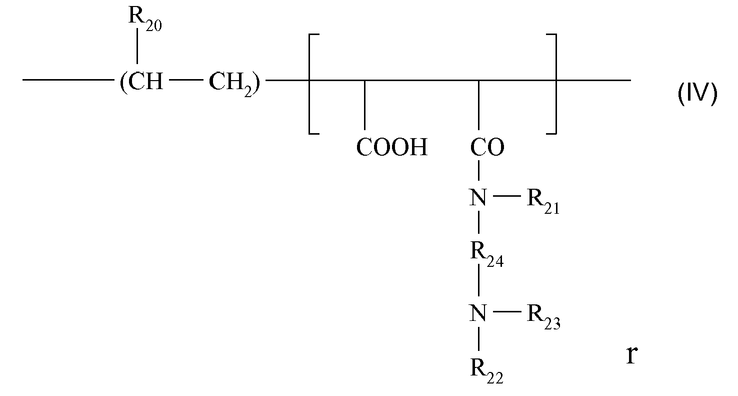 dans laquelle R₂₀ représente un atome d'hydrogène, un radical CH₃O CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂- , -CH₂-CH₂-CH₂- , -CH₂-CH(CH₃)- , R₂₂ ayant les significations mentionnées ci-dessus,
ainsi que les homologues supérieurs de ces radicaux et contenant jusqu'à 6 atomes de carbone.
(9) Des polymères amphotères du type -D-X-D-X choisis parmi:
a) les polymères obtenus par action de l'acide chloracétique ou le chloracétate de sodium sur les composés comportant au moins un motif de formule :
- D-X-D-X-D- (V) où D désigne un radical 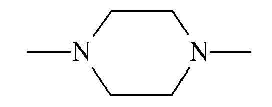 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
b) Les polymères de formule :
-D-X-D-X- (VI)
où D désigne un radical 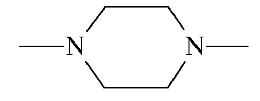 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonction carboxyle ou une ou plusieurs fonctions hydroxyle et bétaïnisées par réaction avec l'acide chloracétique ou du chloracétate de soude.
(10) les copolymères aikyi(C₁-C₅)vinyléther/anhydride maléique modifié partiellement par semiamidification avec une N,N-dialkylaminoalkylamine ou par semiestérification avec une N,N-dialcanolamine.

8. Composition selon la revendication précédente, **caractérisée en ce que** les polymères amphotères sont choisis parmi ceux des familles (1) et (3).

9. Composition selon l'une quelconque des revendications précédentes, **caractérisée en ce que** les polymères amphotères sont choisis parmi les copolymères acide acrylique/chlorure d'acrylamidopropyl triméthyl ammonium les copolymères acide acrylique/chlorure d'acrylamidopropyl triméthyl ammonium/acrylamide, les copolymères comprenant à titre de monomères du chlorure de diméthyldiallylammonium et de l'acide acrylique éventuellement associés à de l'acrylamide.

10. Composition selon l'une quelconque des revendications précédentes, **caractérisée en ce que** les polymères amphotères sont choisis parmi les copolymères acide acrylique/chlorure d'acrylamidopropyl triméthyl ammonium.

11. Composition selon l'une quelconque des revendications précédentes, **caractérisée en ce que** le ou les polymères amphotères représente(nt) de 0,005 à 10% mieux de 0,05 à 5% et encore plus préférentiellement de 0,1 à 2% en poids par rapport au poids total de la composition.

12. Composition selon l'une quelconque des revendications précédentes, **caractérisée en ce qu'**elle comprend des colorants d'oxydation.

13. Composition selon la revendication 12 dans laquelle le colorant d'oxydation est une ou plusieurs bases d'oxydation choisies parmi les paraphénylènediamines, les bis-phénylalkylènediamines, les para-aminophénols, les ortho-aminophénols, les bases hétérocycliques et leurs sels d'addition,et éventuellement un ou plusieurs coupleurs choisis parmi les méta-phénylènediamines, les méta-aminophénols, les méta-diphénols, les coupleurs naphtaléniques, les coupleurs hétérocycliques ainsi que leurs sels d'addition.

14. Composition selon l'une quelconque des revendications précédentes, **caractérisé en ce qu'**elle comprend un ou plusieurs colorants directs choisis parmi des colorants ioniques ou non ioniques, azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

15. Composition selon l'une quelconque des revendications précédentes, **caractérisée en ce qu'**elle comprend à titre d'agents alcalinisants un ou plusieurs composés choisis parmi les agents alcalins minéraux comme l'ammoniaque, les carbonates ou bicarbonates alcalins, les hydroxydes de sodium ou de potassium, les agents alcalins organiques comme les amines organiques dont le pKb à 25°C est inférieur à 12, et de préférence inférieur à 10, encore plus avantageusement inférieur à 6, et les agents alcalins hybrides comme les sels des amines citées précédemment avec des acides comme l'acide carbonique, l'acide chlorhydrique.

16. Composition selon la revendication précédente, **caractérisée en ce que** l'amine organique est une alcanolamine, un acide aminé basique, de préférence la monoéthanolamine.

17. Procédé de coloration ou d'éclaircissement de fibres kératiniques humaines **caractérisé en ce que** l'on applique la composition selon l'une quelconque des revendications précédentes.

18. Dispositif à deux compartiments renfermant dans l'un, une première composition comprenant un ou plusieurs corps gras, un ou plusieurs agents alcalinisants et éventuellement un ou plusieurs colorants d'oxydation et/ou colorants directs ; dans l'autre, une deuxième composition comprenant du peroxyde d'hydrogène ; la première et/ou la seconde composition comprenant un ou plusieurs polymères amphotères ; les compositions des deux compartiments étant destinées à être mélangées pour donner la composition telle que définie dans l'une quelconque des revendications 1 à 16, juste avant l'application sur les fibres kératiniques humaines.

19. Dispositif à trois compartiments renfermant dans l'un, une première composition comprenant un ou plusieurs corps gras ; dans un autre, une deuxième composition comprenant un ou plusieurs agents alcalinisants et éventuellement un ou plusieurs colorants d'oxydation et/ou colorants directs ; et dans le dernier, une troisième composition comprenant du peroxyde d'hydrogène ; la première et/ou la seconde et/ou la troisième composition comprenant un ou plusieurs polymères amphotères ; les compositions des trois compartiments étant destinées à être mélangées pour donner la composition telle que définie dans l'une quelconque des revendications 1 à 16, juste avant l'application sur les fibres kératiniques humaines.
